# EUROPEAN PATENT APPLICATION

(11) **EP 4 647 112 A2**
(43) Date of publication of application: **12.11.2025**
(21) Application number: 25203936.7
(22) Date of filing: 29.03.2021
(51) Int. Cl.: A61N 1/32

(54) **AN ELECTRIC FIELD OR ELECTRIC VOLTAGE DELIVERING ELECTRODE SYSTEM FOR THE TREATMENT OF INTERNAL ORGAN OEDEMA**

(30) Priority: 30.03.2020 EP 20166881; 30.03.2020 US 202063001780 P
(62) Divisional of application: 21714220.7
(71) Applicant: Berlin Heals GmbH, 10719 Berlin (DE)
(72) Inventor: Müller, Johannes, 10717 Berlin (DE)
(74) Representative: Klöckner, Christoph

(57) **Abstract**

The present invention relates to an electric field or electric voltage delivering electrode assembly system for the treatment of internal organ oedema by electrokinetic methods like electro-osmosis and/or electrophoresis by delivering an electric field within a process of treatment of internal organ oedema, wherein the electrode assembly system comprises two electrodes to be positioned at two places on or close to the internal organ or in liquid carrying vessels as part of the organ or in the internal organ. The present invention also relates to the use of such system for inducing an electrokinetic-like effect for treatment of internal organ oedema by means of inducing an electric field. Furthermore, the present invention relates to a process of treatment of internal organ oedema using such system.

## Description

### TECHNICAL FIELD

The present invention relates to an electric field or electric voltage delivering electrode assembly system for the treatment of internal organ oedema by electrokinetic methods like electro-osmosis and/or electrophoresis by delivering an electric field within a process of treatment of internal organ oedema, wherein the electrode assembly system comprises two electrodes to be positioned at two places on or close to the internal organ or in liquid carrying vessels as part of the organ or in the internal organ. The present invention also relates to the use of such system for inducing an electrokinetic-like effect for treatment of internal organ oedema by means of inducing an electric field. Furthermore, the present invention relates to a process of treatment of internal organ oedema using such system.

### PRIOR ART

Primary or secondary disease of an organ, acute or chronic infections or a reduced blood supply to organs are often associated with oedema of the affected organ, which can severely impair the function of the organ. In the heart, this is particularly evident in the form of a restriction of the pumping function, which has an effect on all organs of the body. Thus, internal organ oedema relate to myocardial oedema as described in the following prior art articles but also to kidney oedema or liver oedema to name specific internal organs. Reduced pumping function of the heart typically leads to a congestion of blood in other dependent organs (e.g. liver, kidney), which impresses as oedema in these organs. The extent of the oedema in these organs depends on the severity of the heart dysfunction. Organ oedema can also occur independently of a reduced pumping function of the heart in organ-specific diseases (e.g. diseases of the kidney or liver), such as nephrotic syndrome or inflammation of the liver.

The article "Myocardial Edema on T2-Weighted MRI - New Marker of Ischemia Reperfusion Injury and Adverse Myocardial Remodeling" by Yuko Tada and Phillip C. Yang in http://circres.ahaiournals.org DOI: 10.1161/CIRCRESAHA.117.311494; identifies myocardial oedema as problematic within myocardial infarction patients and suggests new marker therefor. No specific treatment is mentioned.

The article "Why Edema Is a Matter of the Heart" by Matthias G. Friedrich, in http://circimaging.ahaiournals.org DOI: 10.1161/CIRCIMAGING.117.006062 explains the important relationship between myocardial oedema and myocardial infarction and the possibility to differentiate between acute and remote myocardial infarction based on oedema-sensitive CMR (for cardiac magnetic resonance).

In the European Journal of Heart Failure, vol. 2018, edited by the European Society of Cardiology, Thomas M. Gorter answered in a letter to the editor "Myocardial oedema and congestive heart failure: one piece of the puzzle? Reply" on pp827-828, myocardial oedema is mentioned to be an interesting topic for research in view of the link with heart failure.

In "Global myocardial oedema in advanced decompensated heart failure" by Frederik H. Verbrugge et al. in European Heart Journal - Cardiovascular Imaging (2017) 18, pp787- 794 doi:10.1093/ehjci/jew131 it is disclosed that cardiac magnetic resonance (CMR) imaging with quantitative T2 mapping can be used to identify myocardial water content in patients and evaluate the change with decongestive therapy.

Ranjeet M. Dongaonkar et al. shows in "Myocardial microvascular permeability, interstitial oedema, and compromised cardiac function" in Cardiovascular Research (2010) 87, pp331-339 doi:10.1093/cvr/cvq145 the relevance of myocardial oedema as common pathology within instability of the heart. It stipulates that the resolution of myocardial oedema does not restore normal cardiac function. The resolution of myocardial oedema with cardioplegia by avoiding systemic haemodilution is reported.

Maekawa H, Toda G. Nihon Rinsho. (2005;63(1):80-84) have described the negative impact of the storage of water (edema) in the liver for liver function.

In the same way Siddall EC and Radhakrishnan J. (The pathophysiology of edema formation in the nephrotic syndrome. Kidney int. 2012;82(6):635-642. doi:10.1038/ki.2012.180) describe the importance of an oedema in the kidney, as it occurs in the nephrotic syndrome.

As an example of an already known electrode assembly system, WO 2017/021255 A2 describes an implantable direct-current electrode assembly with two implantable electrodes and a control circuit, to which the first and the second electrodes are electrically connected, wherein one of the electrodes is a coil electrode with a maximum length that is pre-determined by the distance between the tricuspid valve and the apex of the right ventricle lying inside the right ventricular cavity between the tricuspid valve and in the right ventricular apex or an area around the apex, whereas the counter-electrode can be a coil electrode for the coronary sinus or a plate electrode that can be attached to the exterior of the left ventricle (epicardium of the left ventricle). Here, the control circuit is particularly designed to establish a potential difference between the two electrodes so that a direct current flows between the two electrodes, wherein the control unit directly sets and feedback controls the desired direct current by means of adjusting the potential difference between the electrodes.

As a further example of prior art, US 2008/0195163 A1 describes an electromedical implantable or extracorporeally applicable device for treating and monitoring organs by allowing healing processes to be excited in diseased organs, wherein the device comprises a power supply unit, a programmable generator and receiver unit which generates and receives electrical microcurrents and electromagnetic power and is connected in a conducting manner to electrodes, a telemetry unit integrated into the generator and receiver unit and provided with a transmitter and a receiver for exchanging data with an extracorporeal device. Therein, the desired current form and frequency are selected and the electrical microcurrents and electromagnetic power are directly controlled by the generator and receiver unit.

However, all of these known prior art devices and methods directly control currents or microcurrents to be applied onto the affected internal organ or liquid carrying vessels for treatment, but none of these known prior art devices and methods even considered the idea of applying an electric field onto the affected internal organ or liquid carrying vessels, and directly controlling the same.

### SUMMARY OF THE INVENTION

Based on the above mentioned prior art documents, it can be seen that several methods are applied to correctly identify myocardial oedema but beside the use of cardioplegia by avoiding systemic haemodilution or directly controlling currents applied onto internal organs or liquid carrying vessels, no reasonable conservative treatments are disclosed in the prior art, in particular there is no method known that specifically is able to reduce the oedema in the myocardium. In this regard, the aqueous solution content of the internal organs and especially of the heart muscle is regulated within very narrow limits, and an increase in the aqueous solution content of only a few percent can significantly impair the function of the heart. It also leads to fibrosis of the heart muscle via molecular mechanisms (increase in TGF-beta).

Therefore, it is an object of the present invention to provide a process and electrode assembly system that is capable to reduce internal organ oedema and especially myocardial oedema with quite simple means. Astonishingly, it has been found that application of an electrical field between two electrodes provided on an internal organ or in liquid carrying vessels in the internal organ over hours immediately starts to reduce the swelling (oedema), e.g. in ten minutes, and further beneficial results appear if the electric field application is maintained up to days, weeks or even long-term (chronically), e.g. three months or a year. Therefore, a bandwidth of treatment period between ten minutes and three months or a year, especially between 12 hours and 7 days is contemplated.

A liquid carrying vessel can be a blood vessel or a vessel of the lymphatic system.

When the process is used to reduce myocardial oedema, then the electrodes are positioned on or near by or in the heart at positions taken from the group comprising inside the right ventricle, inside the coronary sinus, on the outside of the left ventricle and/or on the outside of the right ventricle. Regardless of where the electrodes are placed (for example even subcutaneously or outside the human body with or without skin contact), it is only relevant that the electric field is maintained with electric field lines through the affected organ. In a preferred embodiment, the electrodes, regardless of their technical design, are positioned so that the electric field they generate leads to a reduction in oedema of the corresponding organ. Such positioning is well within the technical skill of a person skilled in the art and can be monitored by techniques known in the art.

When two electrodes, mounted outside of the internal organ, are used, these can be flat electrodes (so called patch electrodes); when an outside mounted electrode is combined with an inside mounted electrode these can be realized as a flat electrode and a coil electrode, respectively. If two inside mounted electrodes are used, they usually are coil electrodes or a combination of a coil electrode and a patch electrode, or two patch electrodes. Then the volume of the treated organ, i.e. the region where the electric field is spanned, is usually smaller than if at least one flat electrode is used, but the density of the electric field is stronger around the cylindrical coil electrode. The electrode according to the invention for reducing oedema of internal organs through spanning an electrical field comprises an electrode support and at least one electrically conductive electrode surface which is embedded in the electrode support, wherein the electrode surface is connected to a control and power supply unit by way of electric lines to charge the electrodes comparable to the electrode plates of a capacitor, wherein the internal organ and any further intervening element as blood vessel and its content, skin if a plate electrode is provided outside the body, provide for the dielectric separating the two conductive plates, with at least one of the plates being electrically isolated.

The predetermined strength of the electric field can be maintained by controlling/regulating the voltage for any given distance of the electrodes. Thus, according to one embodiment of the present invention, the strength of the electric field is directly controlled by setting the voltage to a preset value, preferably to a preset value which has been calculated or at least roughly estimated based on the electroosmotic and/or electrophoretic flow velocity to be achieved e.g. according to the Helmholtz-Smoluchowski relation to generate a specific electrical field strength based on the predetermined distance of the two electrodes to each other. Accordingly, as one embodiment, the control unit is adapted to directly control a strength of an electric field by causing a voltage of a preset value. According to a different embodiment, the strength of the electric field is directly controlled by setting the electric field strength to a preset value. Accordingly, as a different embodiment, the control unit is adapted to directly control a strength of an electric field by causing an electric field strength of a preset value. The electric field can be maintained, in particular, for a time period starting from days, i.e. longer than 24 hours, to weeks and months. Subsequently, it is possible but not necessary to provide switch polarity and charging the electrodes in the opposite way, wherein the polarity of the electrodes can be switched in predetermined time intervals. Such as intervals between ten minutes and three months, or between 12 hours and 7 days.

It may be possible to regulate the electric voltage and/or the electric field indirectly by regulating the current or setting a specific value for the current at a control unit. Using this approach, the specific value for the current would then cause values for the voltage and the electric field largely depending on specific resistance (impedance) and the electrochemical boundary conditions of the myocardial tissue, of the patient, internal organs, medical equipment used as well as possible dynamic variables influencing the resistance (impedance) and/or the resulting voltage and electrical field. Using the current for regulating the strength of the electric field thus causes additional steps of adjusting the current to reach a specific electric field strength.

The application of an electric field creates electrokinetic effects, such as electro-osmosis, electrophoresis or an osmotic like or electrophoretic like effect, wherein electroosmotic and/or electrophoretic flow is caused by the Coulomb force induced by the electric field on net mobile electric charge in a solution. Because the chemical equilibrium between a solid surface and an electrolyte solution typically leads to the interface acquiring a net fixed electrical charge, a layer of mobile ions, known as an electrical double layer or Debye layer, forms in the region near the interface.

The secretion of aqueous solution droplets usually happens at the cathode, however, depending on the composition of the liquid (electrical charge carriers in the liquid) to be removed, the liquid can also be secreted at the anode.

In the case of patch electrodes, it is possible to provide a one-way valve within the patch surface, preferably surrounded by the electrode surface. As a result, the fluid is drained at the point where it has the greatest negative influence on the contact between the electrode surface and the surface of the organic tissue.

Preferably, such a one-way valve is a diaphragm valve having a valve diaphragm.

The process of reduction of the internal oedema applies steps for controlling the electric field between the electrodes according to the present invention wherein the strength of the electric field generated by the electrodes is regulated in such a way that an electric flux (as measure of the electric field through a given surface) is maintained within a predetermined interval for a predetermined surface near the electrode surface.

Due to the selection of such an electric flux, it can be obtained by providing a predetermined voltage for the electrodes, since the strength of an electric field is proportional to the gradient of the voltage at a given distance of the electrodes.

If the electric field is regulated around a predetermined value, a treatment-specific electric flux can be set, which is particularly advantageous since providing a predetermined electroosmotic and/or electrophoretic effect.

The electroosmotic and/or electrophoretic flow is directly related to and depending on the applied voltage and/or electrical field strength. In order to be able to directly influence and control the extent of electroosmotic and/or electrophoretic flow desired for the individual patient, it is thus advantageous to control the voltage and/or electrical field directly. This will make it possible to determine and calculate a desired and adequate value for each individual patient and apply said value from the start of the treatment in a defined way.

Indirect means to control the electroosmotic and/or electrophoretic flow e.g. via setting a specific value for the current is disadvantageous due to the time and additional adaptations necessary to reach a desired voltage and/or field strength. A device, which is only adapted to directly control the current, would have to be adapted to external factors such as medical equipment used as well as factors relating to the patient's status such as weight, water retention or water content within the tissue and others in order to reach a defined electroosmotic and/or electrophoretic flow. Such adaptations will take additional time and are cumbersome and laborious to carry out before the defined treatment can take place.

The system of the present invention, which allows direct control of the voltage and/or the electric field, thus makes direct control of the electroosmotic and/or electrophoretic flow possible. Therefore, the present invention provides an advantageous system, which makes use of the recognition of the present inventors.

This advantageous system is subject-matter of independent claim 1 as filed herewith, and its use as defined in claim 9 as filed herewith.

In further detail, the inventive electrode assembly system for treatment of internal organ oedema by electrokinetic effects, and in further detail electro-osmosis and/or electrophoresis, by delivering an electric field comprises a first electrode, a second electrode and a control unit, wherein the first electrode and second electrode are electrically connected to the control unit, the control unit is adapted to charge the first electrode negatively and the second electrode positively, and the control unit is adapted to directly control a strength of an electric field induced by the first electrode and the second electrode to a preset value for generating a treatment-specific electric flux.

Additionally, the control unit can be configured to (a) allow the preset value of the strength of the electric field to be set and/or entered into the control unit by a user, (b) to automatically control the strength of the electric field to the preset value for the strength of the electric field, and/or (c) to cause and maintain the strength of the electric field at the preset value for the strength of the electric field independently of external influences.

Finally, the already mentioned use of such electrode assembly system is for inducing an electrokinetic-like effect for treatment of internal organ oedema by means of inducing an electric field, wherein the control unit of the electrode assembly is set to a value for the strength of the electric field by a user to directly control an electric field induced by the first electrode and the second electrode to the preset value for generating a treatment-specific electric flux, wherein the preset value of strength of the electric field is preferably caused and maintained by the control unit independent of external influences.

In case of segmented electrodes, e.g. that different parts of a coil electrode are electrically separated one from another or that a flat electrode is separated into to electrically separated electrode surfaces, a control unit can achieve that the electric flux can be concentrated in the adjacent areas of the internal organ to have the necessary electric field maintained in the oedema area of the organ.

Each electrode according to the invention can be used as a positive or negative charge receiving electrode, wherein the cathode is the electrode where the maximum amount of aqueous solution is gathered and conducted away. Therefore, an important reduction effect of the internal oedema happens where the anode provides the liquid-reduced area.

The process of treatment of internal organ oedema can comprise different electrode systems. They always comprise two electrodes and a control unit, wherein the two electrodes can be two patch electrodes, a combination of one patch electrode and one coil electrode, or two coil electrodes. The two electrodes are to be positioned at two places in relation to the internal organ to be treated and the electrodes are connected to the control unit. The control unit is then adapted to provide a voltage generating the electric field with a predetermined electric flux to induce electro-osmosis and/or electrophoresis.

In one embodiment there are provided two patch electrodes, which can be one-surface electrodes or comprises a plurality of separated segments. Then the patch electrodes are mounted on the outer surface of the internal organ, which can be heart, kidney or liver. Mounted can comprise positioning or attaching. It is also possible to position the patch electrodes just subcutaneously or on the outside touching the skin of the patient.

In another embodiment, a patch electrode is combined with a coil electrode, wherein the patch electrode is positioned on the outer surface of the internal organ, wherein the coil electrode is positioned in a liquid carrying vessel of the internal organ. Then the field lines of the electric field are positioned through the organ.

When the internal organ is the heart, the patch electrode is preferably positioned for the heart on the epicardial side of the heart, wherein the coil electrode is positioned for the heart inside the right ventricular cavity. It is only paramount that the electric field lines are extending through the oedemic parts of the heart. The advantage of the pure electric field application is the more flexible positioning of the electrodes. In order to force the field lines cross the myocard, it is preferred to place a coil electrode in one of the ventricles (left or right). The second electrode is not necessarily directly on the internal organ but can be placed, for example, extrapericardially or subcutaneously, or (theoretically) even on the skin, because the electric field also penetrates the lungs, which the current does not like.

When the internal organ is the kidney, the patch electrode is positioned for the kidney on the outer side opposite to the renal artery and renal vein, wherein the coil electrode is positioned inside the renal artery and renal vein or the renal pelvis. As mentioned for the heart, it is also possible for the treatment of an oedemic kidney to place a coil electrode in the renal artery or vein and a patch electrode subcutaneously or even on the skin, since the kidney is directly positioned below the skin. Furthermore, two patch electrodes can be used an each flat side of the kidney. The kidney is then sandwiched by the patch electrodes.

According to one embodiment of the present invention, the electrodes are positioned extracorporally, preferably in physical contact to the skin of a patient. Within the present invention, a place on the outer surface of an internal organ may also include a place on the external skin surface and/or the outer surface of an internal organ may include the external skin surface.

In addition to the aforementioned conditions, myocardial oedema plays a crucial role in the further course of the disease in patients with fresh myocardial infarction or acute myocarditis. A myocardial infarction or myocarditis that cannot be controlled because of myocardial oedema has an increased likelihood of fatal consequences.

Since under acute conditions it is not possible to apply the electrodes directly to an affected internal organ, as this would require a surgical intervention, albeit a minor one, electrodes are envisaged herein that apply the current with its inherent field or the electric field transdermally (with physical contact to the skin).

It is clear to the skilled person that electrodes which are to be positioned extracorporally are structurally different to electrodes to be positioned on internal (intracorporal) organs. Thus, according to one preferred embodiment of the present invention, the electrodes of the present invention and/or the electrode assembly system of the present invention are adapted to allow an extracorporal application thereof. Generally, the size of the electrodes of the present invention may be selected depending on the size of the person to be treated. In a preferred embodiment, the electrodes to be applied extracorporally are patch electrodes.

For any use according to the invention, and in particular for an external or extracorporal use or application of the electrodes according to the present invention, patch electrodes having a size in the range of from 2 by 2 cm (for babies) up to 30 by 40 cm (for adults), and/or a surface area of from 4 cm² to 1200 cm², or any size or surface area in between may be employed. Also, electrodes of different shapes or forms may be used, comprising round, elliptical, square, rectangular and freeform.

In one embodiment of the present invention, the electrodes contacting the skin are electrically conductive allowing electrical current to flow. In one embodiment of the present invention, at least one or all electrodes contacting the skin is/are electrically insulated allowing an electrical field to be generated without any current flow.

To ensure a good transition between the skin and the electrode with as little energy loss as possible, a gel or other liquid with high conductivity may be applied between the extracorporally applied electrode and the skin, similar to substances used for external defibrillation.

Since anti-edematous therapy may require a longer duration of application of the electrodes, one embodiment of the present invention features adhesive electrodes which may be reversibly and directly fixed to the skin surface, preferably wherein the adhesive itself has a favorable resistance behavior. Preferably, the electrically conductive surface of the extracorporal electrode(s) is designed to be deformable so that the electrode(s) can adapt to the body contours.

Generally, the electrically conductive electrode surface may be connected via an electrical energy conducting cable to a device that can generate and deliver the corresponding currents and voltages.

Finally, a process of treatment of internal organ oedema can also use two coil electrodes, wherein the two coil electrodes are positioned in different liquid carrying vessels within the same internal organ. Then the electric field is mainly restricted between the core parts of the organ where the coil electrodes are positioned.

Within this embodiment in an application for the heart, one of the two coil electrodes can be placed in the coronary sinus and the other of the two electrodes can be positioned in the right or left ventricular cavity.

As mentioned above, the internal organ oedema to be treated can be a myocardial oedema or an oedema of the kidney or an oedema of the liver.

The electro-osmosis and/or electrophoresis is generated for a reduction up to a removal of the internal organ oedema. Myocardial oedema, or oedema in other inner organs the excess accumulation of fluid in the myocardial interstitium, develops when there is an imbalance between filtration from the coronary microvasculature, removal of interstitial fluid via lymphatic vessels and epicardial transudation.

The electroosmotic effect comprises an accumulation of oedema fluid at the electrodes to be carried away from the electrodes (transudation). In addition, the effect of electroosmosis and/or electrophoresis is to be found in that the lymphatic system can remove the excess fluid more quickly.

The control unit can be configured to switch the polarity of the electric field in predetermined time intervals. Such predetermined time intervals can comprise intervals between 10 minutes and three months. The entire process can comprise a treatment time of several days up to several months or even chronically. Likewise, the therapy with the electric field can be suspended at any intervals. This means that the device switches off at any interval and then switches on again automatically. The intervals can be a few minutes, hours, days, weeks or months.

Further embodiments of the invention are laid down in the dependent claims.

It is noted that application of an electric field is accompanied by electrolysis generating a pH shift and creation of gas, when the electrodes with electrically conducting surfaces are positioned as explained, e.g. in a liquid vessel or on the internal organ. Such electrolysis does not happen or is very reduced, if at least one of the electrodes is isolated, e.g. sheathed with an isolating material, and the isolation and space between the electrodes act as dielectric. Since the field force is small and the possible electrical current is low and the electrodes are preferably made of platinum or a platinum iridium alloy (or made of a different metal with high positive electrochemical voltage), the effects are limited. It is even so that the shift of the pH towards alkaline can entail positive effects for the patient's regeneration, since a sick inflamed organ or heart frequently shows a slightly lower pH. The gas generation is effected at the anode, which is preferably in the flowing blood (in the blood vessel) or the flowing lymph fluid (in a lymphatic duct). The liquid is capable to dissolve the gas. The generated gas is especially Cl₂, which, immediately after its formation, forms bonds that are physiological and therefore harmless.

The invention further comprises an electrode assembly comprising two electrodes and a control circuit, wherein the first and second electrodes are electrically connected to the control circuit, wherein the control unit is adapted to charge the first electrode negatively and the second electrode positively, especially by direct current or voltage power.

The electrode assembly has preferably a control unit being adapted to switch the polarity of the charged first and second electrodes.

The electrode assembly can comprise two patch electrodes to be positioned opposite one the other of the internal organ so that the electric field generated by the negatively and positively charged electrodes is spanned through the internal organ.

The electrode assembly can have a mixed lay-out with one coil electrode and a patch electrode, wherein the coil electrode is to be positioned inside a liquid vessel of the internal organ or inside the heart and the patch electrode is to be positioned outside of the internal organ so that the electric field generated by the negatively and positively charged electrodes is spanned through the internal organ oedema part.

The electrode assembly can have two coil electrodes to be positioned both inside the heart or in different liquid vessels of the internal organ so that the electric field generated by the negatively and positively charged first and second electrode is spanned through the internal organ oedema part especially between the two liquid vessels.

The control unit of the electrode assembly can be adapted to control the charge of the negatively and positively charged first and second electrodes to control the strength of the electric field at the place of the internal organ oedema over time, i.e. adapt the strength of the electric field during the treatment.

According to a particularly preferred embodiment, the control unit of the electrode assembly is adapted to directly control the strength of the electric field or the amount of the electric voltage during the treatment according to a value set by a user, preferably the control unit automatically controls the strength of the electric field or voltage caused by the electrode assembly in a way that a specified value can be entered by a user which is then caused and maintained by the control unit independent of external influences.

External influences may preferably include differences regarding the inherent resistance (impedance) and the electrochemical boundary conditions of each patient and/or internal organs, equipment used to generate the electric field or voltage or dynamic changes of the resistance (impedance) value during treatment.

The electrode assembly can comprise electrodes in the traditional sense that an electrically conductive surface is in contact with the surrounding tissue or in a liquid vessel of the internal organ to be treated.

The electrode assembly, independent from the fact if it comprises two patch electrodes, two coil electrodes or one coil/one patch electrode can provide a first electrode and a second electrode, wherein one or both can have electrically isolating sheaths. The electrically isolating sheaths are preferably completely encompassing the electrodes until the electric lines leading to the control unit (and also encompassing those). Such electrically isolating sheath can comprise a material taken from the group encompassing silicone, thermoplast, PEEK, PHA and PHB, i.e. use one or more of these and further material but it can also consist of one of these as e.g. silicone or PEEK. Then the effect within the treatment of an internal organ oedema is based on a voltage (electrical field) and a charge applied on the electrode and the dependency of the internal organ, and further body material between the electrodes as body liquids, vessels, organ tissue and possibly skin, alone. The thus directly controlled electric field between the isolated electrodes nevertheless provides a modified electro-osmotic or electrophoretic effect, i.e. an effect of increase of the lymphatic flow, which reduces the oedema of the internal organ, e.g. heart, liver or kidney.

As used herein and in the appended claims, the singular forms "a", "an", and "the" include plural reference unless the context clearly dictates otherwise. Similarly, the words "comprise", "contain" and "encompass" are to be interpreted inclusively rather than exclusively; that is to say, in the sense of "including, but not limited to". Similarly, the word "or" is intended to include "and" unless the context clearly indicates otherwise. The terms "plurality", "multiple" or "multitude" refer to two or more, i.e. 2 or >2, with integer multiples, wherein the terms "single" or "sole" refer to one, i.e. =1. Furthermore, the term "at least one" is to be understood as one or more, i.e. 1 or >1, also with integer multiples. Accordingly, words using the singular or plural number also include the plural and singular number, respectively. Additionally, the words "herein," "above,", "previously" and "below" and words of similar import, when used in this application, shall refer to this application as a whole and not to any particular portions of the application.

Furthermore, certain terms are used for reasons of convenience and are not intended to limit the invention. The terms "right", "left", "up", "down", "under" and "above" refer to directions in the figures. The terminology comprises the explicitly mentioned terms as well as their derivations and terms with a similar meaning. Also, spatially relative terms, such as "beneath", "below", "lower", "above", "upper", "proximal", "distal", and the like, may be used to describe one element's or feature's relationship to another element or feature as illustrated in the figures. These spatially relative terms are intended to encompass different positions and orientations of the devices in use or operation in addition to the position and orientation shown in the figures. For example, if a device in the figures is turned over, elements described as "below" or "beneath" other elements or features would then be "above" or "over" the other elements or features. Thus, the exemplary term "below" can encompass both positions and orientations of above and below. The devices may be otherwise oriented (rotated 90 degrees or at other orientations), and the spatially relative descriptors used herein interpreted accordingly. Likewise, descriptions of movement along and around various axes include various special device positions and orientations.

To avoid repetition in the figures and the descriptions of the various aspects and illustrative embodiments, it should be understood that many features are common to many aspects and embodiments. The description of specific embodiments of the disclosure is not intended to be exhaustive or to limit the disclosure to the precise form disclosed. While the specific embodiments of, and examples for, the disclosure are described herein for illustrative purposes, various equivalent modifications are possible within the scope of the disclosure, as those skilled in the relevant art will recognize. Specific elements of any foregoing embodiments can be combined or substituted for elements in other embodiments. Furthermore, while advantages associated with certain embodiments of the disclosure have been described in the context of these embodiments, other embodiments may also exhibit such advantages, and not all embodiments need necessarily exhibit such advantages to fall within the scope of the disclosure as defined by the appended claims. Omission of an aspect from a description or figure does not imply that the aspect is missing from embodiments that incorporate that aspect. Instead, the aspect may have been omitted for clarity, and in order to avoid prolix description. In this context, the following applies to the rest of this description: If, in order to clarify the drawings, a figure contains reference signs, which signs are not explained in the directly associated part of the description, then it is referred to previous or following description sections. Further, for the reason of lucidity, if in a section of a drawing not all features of a part are provided with reference signs, it is referred to other sections of the same drawing. Like numbers in two or more figures represent the same or similar elements.

The following examples are intended to illustrate various specific embodiments of the present invention. As such, the specific modifications as discussed hereinafter are not to be construed as limitations on the scope of the present invention. It will be apparent to the person skilled in the art that various equivalents, changes, and modifications may be made without departing from the scope of the present invention, and it is thus to be understood that such equivalent embodiments are to be included herein. Further aspects and advantages of the present invention will become apparent from the following description of particular embodiments illustrated in the figures.

### BRIEF DESCRIPTION OF DRAWINGS

Preferred embodiments of the invention are described in the following with reference to the drawings, which are for the purpose of illustrating the present preferred embodiments of a device to execute the process according to the invention and not for the purpose of limiting the same. In the drawings,
- Fig. 1: shows a two internal electrode disposition (two internal coil electrodes) of electrodes in the heart as internal organ;
- Fig. 2: shows a mixed (one internal coil electrode, one external patch electrode) disposition of electrodes in and outside the heart;
- Fig. 3: shows a two external electrodes (external patches with segmented electrodes) disposition of electrodes on external heart surfaces according to the invention during use;
- Fig. 4: shows an electrode according to the invention comprising a one-way valve;
- Fig. 5: shows a two external electrodes (external patches with single electrodes) disposition of electrodes on external kidney surfaces according to the invention during use; and
- Fig. 6: shows a mixed (one internal coil electrode, one external patch electrode) disposition of electrodes in and outside the kidney.

### DESCRIPTION OF PREFFERED EMBODIMENTS

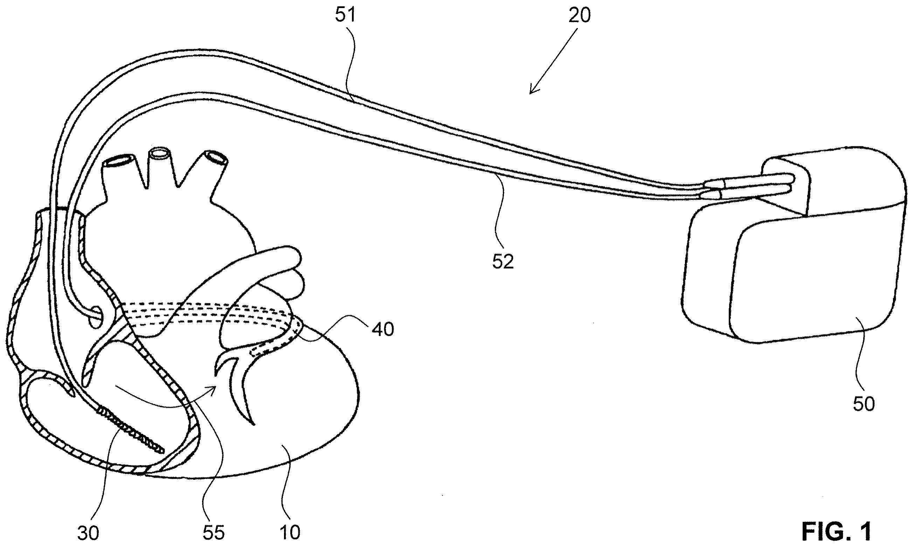

Fig. 1 shows a schematic representation of a heart 10 with an electrode assembly 20 according to a first illustrative embodiment of the invention. The implantable electrode assembly 20 comprises two implantable electrodes 30 and 40 and a control circuit 50, usually arranged in a separate housing in which the battery for the power supply is likewise provided.

The two electrodes 30 and 40 are connected to the control circuit 50 via two single-conductor cables 51 and 52.

The control circuit 50 is designed to establish a potential difference between the two electrodes 30 and 40, such that an electric field is generated between these electrodes 30 and 40.

One electrode 30 is a ventricular electrode, provided for positioning in the right ventricle, and is designed as a coil electrode. It is therefore designated below as a ventricular coil electrode 30. The length of the ventricular coil electrode 30, defined by the one conductive metallic sheath surface or coil surface defining a sheath, is ca. 4 to 10 centimeters and is designed to fill as far as possible the entire length of the right ventricle after passage through the right cardiac tricuspid valve. Here, the ventricular coil electrode 30 is placed loosely into the right ventricle, but it can touch the wall of the right ventricle. To prevent the electrode from falling into the outflow tract of the right ventricle (pulmonary valve), it is anchored actively (by screw) with its tip or passively with barbs in the tip of the right ventricle which hook into the trabecular meshwork of the right ventricle and thus fix the electrode tip.

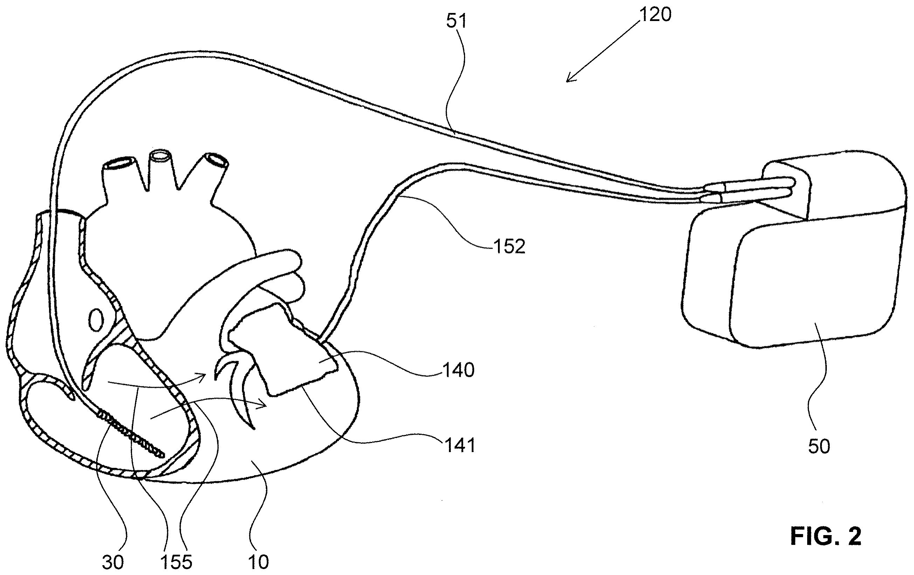

From Fig. 1 and 2, the electrode 30 seems to float freely in the right ventricle. However, this is only apparently the case, because the figures are schematic two-dimensional depictions. Generally, the electrode 30 will nestle on the wall of the ventricle; in the depiction in Fig. 2, this could be the posterior wall, which is not visible there. The electrode 30 is flexible in order to adopt these gentle curvatures, which amount to less than 30 degrees with respect to the longitudinal axis.

The other electrode 40 of Fig. 1 is a coronary sinus electrode, provided for positioning in the coronary sinus, and is likewise designed as a coil electrode. This coronary sinus coil electrode 40 has a smaller diameter than the ventricular coil electrode 30 since it is intended to be advanced far into the coronary sinus in order then to come to lie in the narrowing end region there. This electrode thus lies at a position substantially predefined by the vessel walls, which position the operating surgeon otherwise establishes by advancing it in the longitudinal direction.

When the two electrodes 30 and 40 are connected to a predetermined voltage by the control circuit 50 via the attachment wires or cables 51, 52 insulated from the environment, an electric field is generated with electric field lines shown very schematically according to the arrow 55 through the myocardium. Of course, the electric field lines are directly running from one electrode to the other. In a manner predetermined by the control circuit, the electrode 30 can be the cathode for a predetermined time of between a few minutes and up to chronically, whereby the direction of the electric field is predefined. The control circuit can then switch the polarity of the voltage and change the direction of the electric field after a correspondingly predetermined time, whereby the electrode 40 becomes the cathode. The strength of the electric flux can also change, since the dielectric property of the volume between the two electrodes 30 and 40 can be dependent on the direction of the electric field. In a further illustrative embodiment, the control device controls the electric flux at a uniform predetermined value.

Fig. 2 shows a schematic representation of a heart 10 with an electrode assembly 120 according to a second illustrative embodiment of the invention. The implantable electrode assembly 120 comprises two implantable electrodes 30 and 140 and also a control circuit 50.

Identical features are provided with identical reference signs, similar features with correspondingly similar reference signs.

The control circuit 50 can be designed in the same way as described in Fig. 1. The two electrodes 30 and 140 are also connected to the control circuit 50 via two single-conductor cables 51 and 52.

The control circuit 50 is also designed here to establish an electric field between the two electrodes 30 and 140, such that an electro-osmotic and/or electrophoretic effect can exist between these electrodes 30 and 140 for a predetermined time of several minutes, e.g. 5 minutes, to several days, e.g. 3 days or even chronically.

One electrode 30 is once again a ventricular electrode, provided for positioning in the right ventricle, and is designed as a coil electrode. It is therefore also designated here as a ventricular coil electrode 30. The length of the ventricular coil electrode 30, defined by the one conductive metallic sheath surface or coil surface defining a sheath, is ca. 4 to 10 centimeters and is designed to fill as far as possible the entire length of the right ventricle in the longitudinal axis after passage through the right cardiac valve (tricuspid valve). Here, the ventricular coil electrode 30 is placed loosely into the right ventricle, is passively anchored at the distal end and can bear on the wall of the ventricle or on the septum. To prevent the electrode from falling into the outflow tract of the right ventricle (pulmonary valve), it is anchored actively (by screw) with its tip or passively with barbs in the tip of the right ventricle.

The other electrode 140 is a surface electrode (patch electrode), provided for positioning on the epicardium, the pericardium or close to the epicardium (e. g. even subcutaneously). It can be designed, for example, according to the teaching of US 2008/0195163 A1. This surface electrode 140 is applied to the left side of the myocardium, epicardially opposite the right ventricle.

When the two electrodes 30 and 140 are subjected to a potential difference by the control circuit 50 via the attachment wires or cables 51, 52 insulated from the environment, an electric field is generated with field lines very schematically in the direction according to the arrows 155 through the myocardium. These electric field lines are symbolized here by two arrows which essentially show the approximate direction of the field lines, since the field lines emerge and fan out from a substantially longitudinally dimensional face of the substantially longitudinally oriented surface of the coil electrode 30 toward the surface electrode 140 and thus sweeps across a fan. Seen physically, the main portion of the electric field lines are inside a prism; that is to say proceeding from an edge (of the prism) to its base on the patch electrode.

A prism is by definition a geometric body whose side edges are parallel and of equal length and which has a polygon as base. It arises from parallel displacement of a plane polygon along a straight line not lying in this plane and is therefore a special polyhedron. Here, the straight line is predefined by the longitudinal axis of the coil electrode 30, and the polygon is a triangle with the apex at the coil electrode 30 and with a base that corresponds to the width of the surface electrode (patch electrode) 140. If these side edges 141 of the surface electrode 140 do not come to lie parallel to the orientation of the coil electrode, it is a rotated prism. In all cases, the two electrodes 30 and 140 define a not inconsiderable spatial body, which guarantees that the electric field spans through a likewise not inconsiderable sub region of the left cardiac muscle and to a slightly lesser extent also of the right cardiac muscle. Describing the geometry of the body through which the electric field has a substantive strength as a prism is an approximation, since it can be assumed from this that the electrode does not float freely but is instead passively fixed at its distal tip and then bears on the wall of the ventricle. The boundary lines of the body are then certainly not straight but curved, and the defined body is then obtained only approximately as a prism. Of importance, however, is the narrow "edge" on the one side formed by the coil electrode, and the "broad bottom face" on the other side, which is formed by the patch electrode.

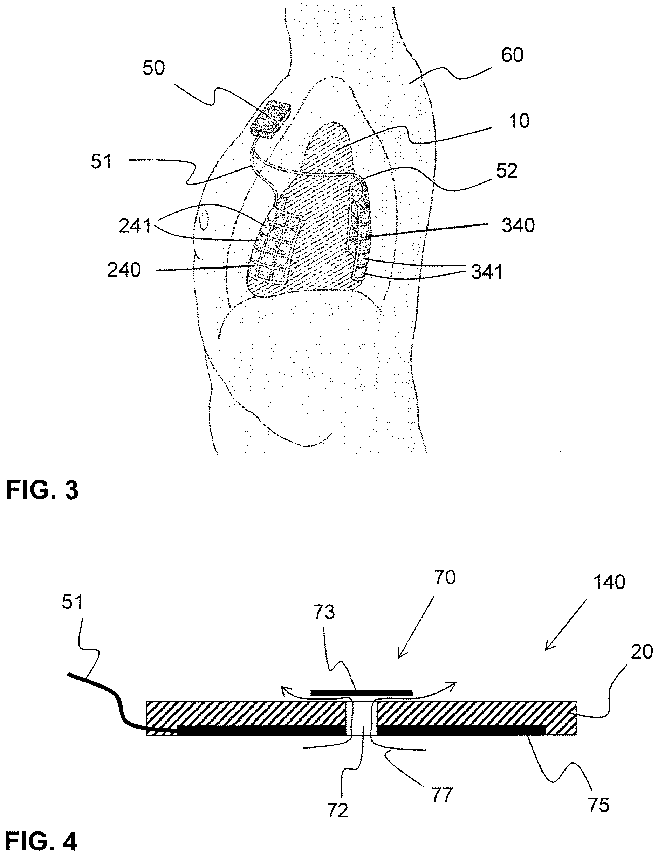

Fig.3 shows two patch electrodes 240 and 340, which are connected with lines 51 and 52 to a control and power supply unit 50. Here, each patch electrode 240 and 340 as such is a segmented electrode 240 or 340. This means, each electrode 240 or 340 is or comprises a plurality of electrode segments 241 or 341, which are shown as smaller rectangles in Fig. 3.

Although the organ 10 can be a heart, it is also possible that the organ 10 is a kidney with applied electrodes 240 and 340. In other embodiments, it could be a liver.

The electrode 240 or 340 optionally comprises at least one one-way valve 70, which essentially comprises an opening 72 and a diaphragm 73 covering the opening 72 on the far side of the internal organ. A schematic sectional view of the one-way valve 70 is depicted in Fig. 4. The diaphragm is made from silicone, for example. The one-way valve 70 is situated within the electrode surface 75.

The apparatus as described in connection with Fig. 1, 2 or 3 provides a constant electric field with defined polarity for a given time as hours or days applied to the tissue of an internal organ, here the heart. This electric field acts immediately or at least extremely fast on the internal organ, here the heart.

Extremely fast means that the first signs of improvement starts to appear within minutes after the electric field is applied. Irrespective of the fact that the patient describes a better feeling of well-being, echocardiography can be used to objectify a reduction in the size of the ventricle very quickly as the first positive sign. In this short period of time, this immediate improvement cannot be explained by molecular biological processes in the heart musculature; the improvement is due to electro-osmosis, a process by which an aqueous solution, such as water for example, is transported osmotically (used, for example, to dry damp walls or in cosmetics), and/or electrophoresis, a process by which charged components, such as proteins, electrolytes, or molecules, are transported.

It can be assumed that diseased organs are always also inflamed organs and inflammation is always associated with oedema. In relation to the heart, this means that there is an intra- and/or extracellular excess of fluid (oedema), which causes the heart muscles to swell and limits their pumping function. By applying an electric field or voltage, an electrokinetic-like effect, i.e. an osmotic-like and/or electrophoretic-like effect is induced, by which aqueous solution is extracted from the heart muscles, which was before enlarged and bloated by oedema and flow in the lymphatic system is increased and the cardiac interstitium is drained.

Such drainage of the heart muscle, i.e. the reduction of myocardial oedema, might be difficult to monitor in living organisms, but the inventors of the present invention have found that the subsequent improvement of the heart function is substantially based on the effect known as electroosmosis and/or electrophoresis.

This effect is transferable to other internal organs in which the intra- and/or extracellular excess of fluid (oedema) leads to restricted cell function and thus reduces organ function as a whole. In general, it is pointed out that the described liquid drainage of oedema-afflicted organs or vessels can also be referred to as lymphatic drainage since the lymphatic vessels are one of the transport routes, or the most important transport route, that remove the oedema and are stimulated by electroosmosis.

This presently described function is based on the insight, that the human body is a so-called ion conductor. The electric field mainly causes ion movement (electrokinesis), i.e. the migration of negatively charged anions (e.g. Cl-, CO₂- etc) to the anode and of positively charged cations (e.g. Na+. Mg++.etc) to the cathode. Electro-chemical reactions occur on metals (electrodes, but also metallic foreign bodies). A migration of protein fractions, i.e. electrophoresis, and a shift of aqueous solution in the direction of the cathode, i.e. electro-osmosis, takes place in the applied electric field, i.e. transudation is caused.

Also, as is known, the smallest vessels that regulate liquid content in myocardial tissue are the capillaries. In this regard, it is pointed out that the capillaries and other vessels as well are lined with a layer called glycocalyx, which controls the permeability of the vessel walls to fluid and other substances and exhibits a strong negative charge when in a healthy state.

However, in case this negative charge is disturbed, for example by disease or the like, the permeability of the vessels can increase and more fluid and other substances can leak out of the vessel, which then results in the occurrence of oedema of the myocardium. In addition to the above described effect, the charge of the glycocalyx can be restored by the applied electric field, thereby supporting the reduction of the oedema and consequently the improvement of cardiac function.

The above described effect is as such independent from the application with two internal coil electrodes, one coil electrode and one patch electrode or two patch electrodes applied on opposite parts of the internal organ like the on the left and the right ventricle.

This can be achieved with electrodes in the blood vessel system of the organ in question or in the lymphatic system.

This can further be achieved by exposing the affected organ or parts of the organ and the blood vessel system, in particular the capillaries of the blood vessel system, to a suitable electric field or current with an inherent electric field generated by any electrode configuration as described herein. The lymphatic system, as part of the affected organ, is thus simultaneously exposed to the field or current and responds by an increased and accelerated lymphatic flow.

Although the drawings Fig. 1 to 4 only show the heart in the application, similar coil electrodes can be used for the treatment of a liver and/or a kidney. They can be used in blood vessels or the lymphatic system. Flat electrodes can be positioned on or near the liver or kidney with the flat electrodes on mainly opposite sides of the organ, so that the electric field and its field lines passes through the organ. This can also be done subcutaneously or from the exterior of the human body.

As an example of the clinical success of the above described osmotic-like and/or electrophoretic effect achieved by directly controlling an electric field applied onto the oedema-afflicted organs or vessels, the following results have been reported (Kosevic, D et al. (2021). Cardio-microcurrent device for chronic heart failure: first-in-human clinical study. ESC heart failure. 10.1002/ehf2.13242).

The average of patients included in the study reported therein is a New York Heart Association (NYHA) Class III non-ischemic patient in the age group of 29 to 67 years, with a body mass index of 22.5 to 35.9 and a history of heart failure, and in particular with a significantly reduced left ventricular ejection fraction (LVEF) and a 6 minute walk under about 250 m. The "6 min walk test" or "6MWT" has been developed by the American Thoracic Society as a reliable indicator in the form of a sub-maximal exercise test for assessing aerobic capacity and endurance, wherein the walking distance covered over a time of 6 minutes by the patient is used as the outcome by which to compare changes in performance capacity. Here, the average patient (as described before) achieved between ~170 and ~250 m at hospitalization, between ~350 and ~450 m after 14 days, and between ~370 and ~470 m after 6 months of device use, and, furthermore, the average patient's classification according to the NYHA improved to a significantly less critical class after this time period.

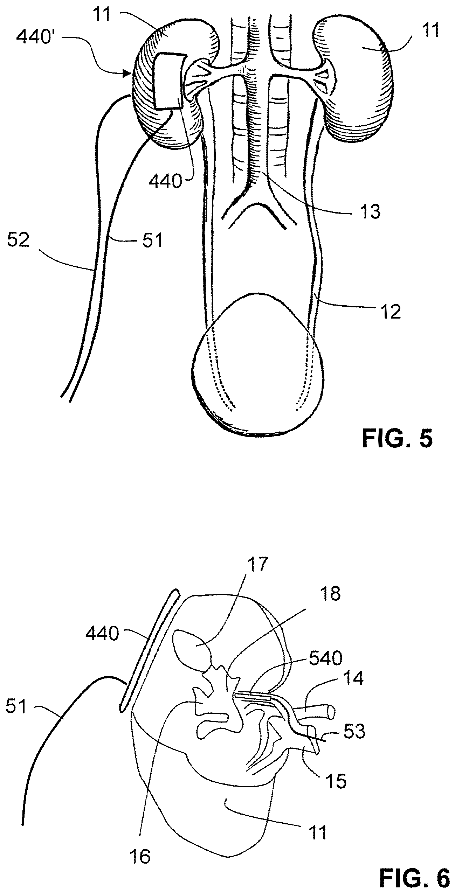

Fig. 5 shows a two external electrodes (external patches with single electrodes) disposition of electrodes on external kidney surfaces according to the invention during use. There are two kidneys 11 with a symbolic central aorta or vena renalis 13. Ureters 12 connect the kidneys 11 with the bladder of the person. A patch electrode 440 is positioned on the outside of one kidney 11. A second patch electrode 440' (with an identical outlay to the first patch electrode 440) is positioned on the opposite side of the kidney 11. Therefore, the core part of the kidney with its renal pyramids 17, renal calix 16 and the renal pelvis 18 is positioned between the two patches 440 and patches 440'.

The flat electrode patches 440 and 440' are connected with a control unit 50, not shown in Fig. 5, via connection lines 51 and 52, respectively. The supply lines 51 and 52 provide an electric field between the flat electrode patches 440 and 440' wherein the electric flux through the mentioned parts of the kidney then effectively reduces the oedema through electro-osmotic and/or electrophoretic effects. The electrode patches 440 and 440' are shown as single electrodes but can also be segmented electrodes as shown in Fig. 3.

Fig. 6 shows a mixed (one internal coil electrode, one external patch electrode) disposition of electrodes in and outside the kidney. The kidney 11 is shown with its renal pyramids 17, renal calix 16 and the renal pelvis 18. The aorta renalis 14 and vena renalis 15 are shown as well. A first external electrode 440 is connected via line 51 to a control unit 50 (not shown). A renal coil electrode 540 is positioned in the vena renalis 15 to allow an electric field between this electrode 540 and said external patch electrode 440. The connection of the renal coil electrode 540 to the control unit 50 is effected via the catheter line 53 used to position the renal coil electrode 540. The coil electrode could in other embodiments also positioned in the renal lymphatic system.

Within the electrode assembly 20, 120, the lines 51, 52 and/or 53 are usually electrically isolated, e.g. in a catheter or in an isolating sheath. The two electrodes, independent from the fact if they are two patch electrodes 140, 240, 340, 440, 440' especially non-segmented and having one single electrode surface each, two coil electrodes 30, 40 or one coil/one 5 patch electrode 30/140,440/540 can provide a first electrode and a second electrode which one or both can have electrically isolating sheaths. The electrically isolating sheaths are preferably completely encompassing the electrodes until the electric lines 51, 52 or 53 leading to the control unit 50 (and also encompassing these elements). Such electrically isolating sheath can comprise a material taken from the group encompassing silicone, thermoplast, PEEK, 10 PHA and PHB, i.e. use one or more of these and further material but it can also consist of one of these as e.g. silicone or PEEK alone. Then the effect of treatment of internal organ oedema is based on a voltage and a charge applied on the electrode and the dependency of the internal organ, and further body material between the electrodes as body liquids, vessels, organ tissue, and possibly skin), i.e. presetting and directly controlling the electric field between the isolated electrodes alone provides the desired electro-osmotic and/or electrophoretic effect which reduces the oedema of the internal organ, e.g. heart, liver or kidney.

The present invention further relates to the following items:
Item 1:
   An electrode assembly system 20, 120 for treatment of internal organ oedema by electro-osmosis and/or electrophoresis by delivering an electric field, the system comprising a first electrode 30; 240; 440, a second electrode 40, 140; 340; 440', 540, and a control unit 50, wherein the first electrode 30; 240; 440 and second electrode 40, 140; 340; 440', 540 are electrically connected to the control unit 50, characterized in that the control unit 50 is adapted to charge the first electrode 30; 240; 440 negatively and the second electrode 40, 140; 340; 440', 540 positively, and the control unit 50 is adapted to directly control a strength of an electric field induced by the first electrode 30; 240; 440 and the second electrode 40, 140; 340; 440', 540 to a preset value for generating a treatment-specific electric flux.
Item 2:
   The electrode assembly system 20, 12 according to the item 1, wherein the control unit 50 is adapted to switch the polarity of the charged first and second electrodes.
Item 3:
   The electrode assembly system 20, 120 according to the item 1 or 2, wherein the first electrode is a patch electrode 240, 440 and the second electrode is a patch electrode 340, 440' to be positioned opposite one the other of the internal organ 10, 11 so that the electric field generated by the negatively and positively charged first and second electrodes is spanned 55, 155 through the internal organ 10, 11.
Item 4:
   The electrode assembly system 20, 120 according to the item 1 or 2, wherein the first electrode is a coil electrode 30, 540, and the second electrode is a patch electrode 140, 440, wherein the first electrode 30, 540 is to be positioned inside a liquid vessel of the internal organ 10,11 and the second electrode 140,440 is to be positioned outside of the internal organ 10,11 so that the electric field generated by the negatively and positively charged first and second electrodes is spanned through the internal organ oedema part.
Item 5:
   The electrode assembly system 20, 120 according to item 1 or 2, wherein the first electrode is a coil electrode 30 and the second electrode is a coil electrode 40, wherein the first electrode 30 is to be positioned inside a liquid vessel of the internal organ 10 and the second electrode 40 is to be positioned inside a liquid vessel of the internal organ 10 so that the electric field generated by the negatively and positively charged first and second electrodes is spanned through the internal organ oedema part.
Item 6:
   The electrode assembly system 20, 120 according to any one of items1-5, wherein the control unit 50 is adapted to control the charge of the negatively and positively charged first and second electrodes to control the strength of the electric field at the place of the internal organ oedema.
Item 7:
   The electrode assembly system (20, 120) according to any one of items 1 to 6, wherein
   the control unit (50) is configured to allow the preset value of the strength of the electric field to be set and/or entered into the control unit (50) by a user,
   the control unit (50) is configured to automatically control the strength of the electric field to the preset value for the strength of the electric field , and/or
   the control unit (50) is configured to cause and maintain the strength of the electric field at the preset value for the strength of the electric field independently of external influences.
Item 8:
   The electrode assembly system (20, 120) according to any one of items 1 to 7, wherein the first electrode and the second electrode have each an electrically isolating sheath, preferably wherein
   the electrically isolating sheath comprises a material taken from the group encompassing silicone, thermoplast, PEEK, PHA and PHB.
Item 9:
   The electrode assembly system (20, 120) according to any one of items 1 to 8, wherein the electrodes are positioned extracorporally, preferably wherein the electrodes are positioned in physical contact with the external skin surface, more preferably wherein the electrodes can be attached or adhered to the skin surface.
Item 10:
   Use of an electrode assembly system (20, 120) according to any one of items 1 to 9 for inducing an osmotic-like and/or electrophoretic-like effect for treatment of internal organ oedema by means of inducing an electric field, wherein the control unit (50) of the electrode assembly is set to a value for the strength of the electric field by a user to directly control an electric field induced by the first electrode (30; 240; 440) and the second electrode (40, 140; 340; 440', 540) to the preset value for generating a treatment-specific electric flux, wherein the preset value of strength of the electric field is preferably caused and maintained by the control unit independent of external influences.
Item 11:
   A process of treatment of internal organ oedema using an electrical field delivering electrode system comprising two patch electrodes and a control unit, wherein the two patch electrodes are to be positioned at two places on the outer surface of the internal organ, wherein the two patch electrodes are connected to the control unit and wherein the control unit is adapted to provide an electric field between the electrodes to induce electro-osmosis and/or electrophoresis.
Item 12:
   A process of treatment of internal organ oedema using an electrical field delivering electrode system comprising a patch electrode, a coil electrode and a control unit, wherein the patch electrode is positioned on the outer surface of the internal organ, wherein the coil electrode is positioned in a liquid carrying vessel of the internal organ, the patch electrode and the coil electrode are connected to the control unit and the control unit is adapted to provide an electric field between the two electrodes to induce electro-osmosis and/or electrophoresis, wherein optionally the patch electrode is positioned for the heart on the epicardial side of the heart and wherein the coil electrode is positioned for the heart inside the ventricular cavity.
Item 13:
   A process of treatment of internal organ oedema using an electric field delivering electrode system comprising two coil electrodes, wherein the two coil electrodes are positioned in different liquid carrying vessels within the internal organ, the two coil electrodes are connected each to the control unit and the control unit is adapted to deliver an electric field between the two electrodes to induce electro-osmosis and/or electrophoresis, wherein optionally one of the two coil electrodes is placed in the coronary sinus and the other of the two electrodes is positioned in the right or left ventricular cavity.
Item 14:
   The process of treatment of internal organ oedema according to any one of claims 11 to 13, wherein the internal organ oedema is a myocardial oedema or an oedema of the kidney or an oedema of the liver, and wherein the electro-osmosis and/or electrophoresis is generated for a reduction up to a removal of the internal organ oedema and wherein the electroosmotic and/or electrophoretic effect comprises an accumulation of oedema fluid at the electrodes to be carried away from the electrodes and/or an increased lymphatic flow in the lymphatic vessels induced by the electric field.
Item 15:
   The process of treatment of internal organ oedema according to any one of the items 11 to 14, wherein the control unit is configured to switch the polarity of the electrodes in predetermined time intervals, preferably wherein the predetermined time intervals comprise intervals between ten minutes and three months, especially between 12 hours and 7 days.

**LIST OF REFERENCE SIGNS**

| | | | |
|---|---|---|---|
| 10 | heart (internal organ) | 60 | person |
| 11 | kidney (internal organ) | 70 | patch electrode surface |
| 12 | ureter | 72 | opening |
| 13 | aorta or vena renalis | 73 | diaphragm valve |
| 14 | aorta renalis | 75 | electrode surface |
| 15 | vena renalis | 77 | flow of aqueous solution |
| 16 | renal calix | 120 | electrode assembly |
| 17 | renal pyramid | 140 | external patch electrode |
| 18 | renal pelvis | 141 | edge of the surface electrode |
| 20 | electrode assembly | 152 | single-conductor supply line |
| 30 | ventricular coil electrode | 155 | arrow indicating general direction of electric field lines |
| 40 | coil electrode for coronary sinus | 240 | first external patch electrode |
| 50 | control circuit | 340 | second external patch electrode |
| 51 | single-conductor supply line | | |
| 52 | single-conductor supply line | 440 | first external patch electrode |
| 53 | renal catheter line | 440' | second external patch electrode |
| 55 | arrow indicating general direction of electric field lines | 540 | renal coil electrode |

## Claims

1. An electrode assembly system (20, 120) for treatment of internal organ oedema by electro-osmosis and/or electrophoresis by delivering an electric field, the system comprising a first electrode (30; 240; 440),
a second electrode (40, 140; 340; 440', 540), and
a control unit (50),
wherein the first electrode (30; 240; 440) and second electrode (40, 140; 340; 440', 540) are connected to the control unit (50) and are to be positioned at two places in relation to the internal organ,
**characterized in that**
the control unit (50) is adapted to directly control a strength of an electric field induced by the first electrode (30; 240; 440) and the second electrode (40, 140; 340; 440', 540) to a preset value for generating a treatment-specific electric flux, wherein the control unit is adapted to maintain the strength of the electric field at the preset value by controlling or regulating a voltage between the first electrode (30; 240; 440) and the second electrode (40, 140; 340; 440', 540) for any given distance between the electrodes.

2. An electrode assembly system (20, 120) according to claim 1, wherein each of the first electrode (30; 240; 440) and second electrode (40, 140; 340; 440', 540) is used as a positive or negative charge receiving electrode.

3. The electrode assembly system (20, 120) according to claim 2, wherein the control unit (50) is adapted to switch the polarity of the charged first and second electrodes.

4. The electrode assembly system (20, 120) according to any of claims 1-3, wherein the first electrode is a patch electrode (240, 440) and the second electrode is a patch electrode (340, 440') to be positioned opposite one the other of the internal organ (10, 11) so that the electric field generated by the first and second electrodes is spanned (55, 155) through the internal organ (10, 11).

5. The electrode assembly system (20, 120) according to any of claims 1-3, wherein the first electrode is a coil electrode (30, 540), and the second electrode is a patch electrode (140, 440), wherein the first electrode (30, 540) is to be positioned inside a liquid vessel of the internal organ (10,11) and the second electrode (140,440) is to be positioned outside of the internal organ (10,11) so that the electric field generated by the first and second electrodes is spanned through the internal organ oedema part.

6. The electrode assembly system (20, 120) according to any of claims 1-3, wherein the first electrode is a coil electrode (30) and the second electrode is a coil electrode (40), wherein the first electrode (30) is to be positioned inside a liquid vessel of the internal organ (10) and the second electrode (40) is to be positioned inside a liquid vessel of the internal organ (10) so that the electric field generated by first and second electrodes is spanned through the internal organ oedema part.

7. The electrode assembly system (20, 120) according to any one of claims 1 to 6, wherein the control unit (50) is adapted to control the charge of the first and second electrodes to control the strength of the electric field at the place of the internal organ oedema.

8. The electrode assembly system (20, 120) according to any one of claims 1 to 7, wherein the control unit (50) is configured to allow the preset value of the strength of the electric field to be set and/or entered into the control unit (50) by a user,
the control unit (50) is configured to automatically control the strength of the electric field to the preset value for the strength of the electric field , and/or
the control unit (50) is configured to cause and maintain the strength of the electric field at the preset value for the strength of the electric field independently of external influences.

9. The electrode assembly system (20, 120) according to any one of claims 1 to 8, wherein the first electrode and the second electrode have each an electrically isolating sheath, preferably wherein the electrically isolating sheath comprises a material taken from the group encompassing silicone, thermoplast, PEEK, PHA and PHB.

10. The electrode assembly system (20, 120) according to any one of claims 1 to 9, wherein the electrodes are positioned extracorporally, preferably wherein the electrodes are positioned in physical contact with the external skin surface, more preferably wherein the electrodes can be attached or adhered to the skin surface.

11. Use of an electrode assembly system (20, 120) according to any one of claims 1 to 10 for inducing an osmotic-like and/or electrophoretic-like effect for treatment of internal organ oedema by means of inducing an electric field, wherein the control unit (50) of the electrode assembly is set to a value for the strength of the electric field by a user to directly control an electric field induced by the first electrode (30; 240; 440) and the second electrode (40, 140; 340; 440', 540) to the preset value for generating a treatment-specific electric flux, wherein the preset value of strength of the electric field is preferably caused and maintained by the control unit independent of external influences.

12. A process of treatment of internal organ oedema using an electrical field delivering electrode system comprising two patch electrodes and a control unit, wherein the two patch electrodes are to be positioned at two places on the outer surface of the internal organ, wherein the two patch electrodes are connected to the control unit and wherein the control unit is adapted to provide an electric field between the electrodes to induce electro-osmosis and/or electrophoresis.

13. A process of treatment of internal organ oedema using an electrical field delivering electrode system comprising a patch electrode, a coil electrode and a control unit, wherein the patch electrode is positioned on the outer surface of the internal organ, wherein the coil electrode is positioned in a liquid carrying vessel of the internal organ, the patch electrode and the coil electrode are connected to the control unit and the control unit is adapted to provide an electric field between the two electrodes to induce electro-osmosis and/or electrophoresis, wherein optionally the patch electrode is positioned for the heart on the epicardial side of the heart and wherein the coil electrode is positioned for the heart inside the ventricular cavity.

14. A process of treatment of internal organ oedema using an electric field delivering electrode system comprising two coil electrodes, wherein the two coil electrodes are positioned in different liquid carrying vessels within the internal organ, the two coil electrodes are connected each to the control unit and the control unit is adapted to deliver an electric field between the two electrodes to induce electro-osmosis and/or electrophoresis, wherein optionally one of the two coil electrodes is placed in the coronary sinus and the other of the two electrodes is positioned in the right or left ventricular cavity.

15. The process of treatment of internal organ oedema according to any one of claims 12 to 14, wherein the internal organ oedema is a myocardial oedema or an oedema of the kidney or an oedema of the liver, and wherein the electro-osmosis and/or electrophoresis is generated for a reduction up to a removal of the internal organ oedema and wherein the electroosmotic and/or electrophoretic effect comprises an accumulation of oedema fluid at the electrodes to be carried away from the electrodes and/or an increased lymphatic flow in the lymphatic vessels induced by the electric field.

16. The process of treatment of internal organ oedema according to any one of the claims 12 to 15, wherein the control unit is configured to switch the polarity of the electrodes in predetermined time intervals, preferably wherein the predetermined time intervals comprise intervals between ten minutes and three months, especially between 12 hours and 7 days.
